# EUROPEAN PATENT APPLICATION

(11) **EP 3 245 955 A1**
(43) Date of publication of application: **22.11.2017**
(21) Application number: 16202474.9
(22) Date of filing: 06.12.2016
(51) Int. Cl.: A61B 10/00

(54) **TISSUE SAMPLE RECEPTACLE**

(30) Priority: 16.05.2016 US 201662337161 P; 02.12.2016 US 201615368244
(71) Applicant: GTI Services, LLC, Stafford, TX 77477 (US)
(72) Inventor: NOSAVILLE, Yury, Stafford, TX 77477 (US); SORKIN, Felix, Stafford, TX 77477 (US); NOSAVILLE, Ross, Stafford, TX 77477 (US)
(74) Representative: Bond, Christopher William

(57) **Abstract**

A tissue sample receptacle includes a fulcrum body. The fulcrum body is positioned to allow a tissue sample collector to be broken off within the tissue sample receptacle. The fulcrum body provides a fulcrum against which the tissue sample collector may be bent. The fulcrum body may be coupled to the sidewall of the tissue sample receptacle. The fulcrum body may be removable from, may be permanently coupled to, or may be formed integrally with the sidewall.

## Description

This application claims priority from: U.S. provisional application number 62/337,161, filed May 16, 2016; and, U.S. non-provisional application number 15/368,244, filed 2 December 2016.

### Technical Field/Field of the Disclosure

The present disclosure relates to medical devices, and specifically to tissue sample bottles for collecting medical samples.

### Background of the Disclosure

In certain medical diagnostic procedures, tissue samples may be obtained from a patient. Tissue samples are traditionally collected by a tissue sample collector such as a brush, spatula, or broom-like device. The tissue samples may be deposited in a sample container such as a sealable bottle. The sample bottle is traditionally at least partially filled with a fluid, for example, a diagnostic reagent or a stabilizer for storing the tissue sample.

### Summary

The present disclosure provides for a tissue sample receptacle. The tissue sample receptacle may include a container and a fulcrum body positioned on the interior of the container.

The present disclosure also provides for a method. The method may include providing a tissue sample receptacle. The tissue sample receptacle may include a container and a fulcrum body positioned on the interior of the container. The method may include inserting the tissue sample collector into the tissue sample receptacle. The method may include applying a force against an upper end of the tissue sample collector such that the tissue sample collector is bent between the fulcrum body and the sidewall. The method may include breaking the tissue sample collector.

### Brief Description of the Drawings

The present disclosure is best understood from the following detailed description when read with the accompanying figures. It is emphasized that, in accordance with the standard practice in the industry, various features are not drawn to scale. In fact, the dimensions of the various features may be arbitrarily increased or reduced for clarity of discussion.
FIG. 1 depicts a tissue sample receptacle consistent with at least one embodiment of the present disclosure.
FIGS. 2A, 2B depict a cross section view of a tissue sample receptacle consistent with at least one embodiment of the present disclosure.
FIG. 3 depicts a top view of a tissue sample receptacle consistent with at least one embodiment of the present disclosure.
FIG. 4 depicts a cross section view of a tissue sample receptacle consistent with at least one embodiment of the present disclosure.
FIG. 5 depicts a cross section view of a tissue sample receptacle consistent with at least one embodiment of the present disclosure.

### Detailed Description

It is to be understood that the following disclosure provides many different embodiments, or examples, for implementing different features of various embodiments. Specific examples of components and arrangements are described below to simplify the present disclosure. These are, of course, merely examples and are not intended to be limiting. In addition, the present disclosure may repeat reference numerals and/or letters in the various examples. This repetition is for the purpose of simplicity and clarity and does not in itself dictate a relationship between the various embodiments and/or configurations discussed.

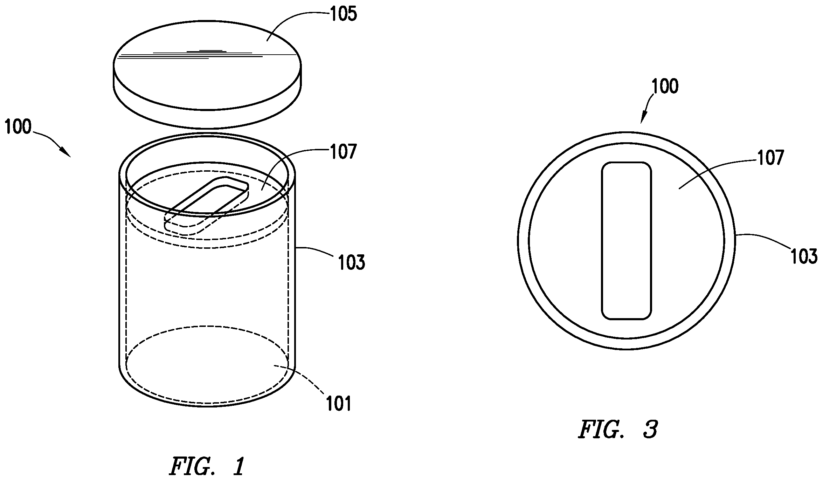

As depicted in FIGS. 1-3, tissue sample receptacle 100 may be a sealable container or bottle. Although depicted as being cylindrical, any cross sectional shape may be used without deviating from the scope of this disclosure. Tissue sample receptacle 100 may include bottom 101 and sidewall 103. Although described herein and depicted as a bottle, tissue sample receptacle 100 may be a vial or tube without deviating from the scope of this disclosure. In some embodiments, tissue sample receptacle 100 may be a bag. In some embodiments, tissue sample receptacle 100 may not include bottom 101. In some embodiments, removable cap 105 may be mechanically coupleable to sidewall 103 of tissue sample receptacle 100. In some embodiments, cap 105 may form an air or liquid tight seal against sidewall 103 of tissue sample receptacle 100. In some embodiments, cap 105 may mechanically couple to sidewall 103 by, for example and without limitation, a snap fit or threaded connection.

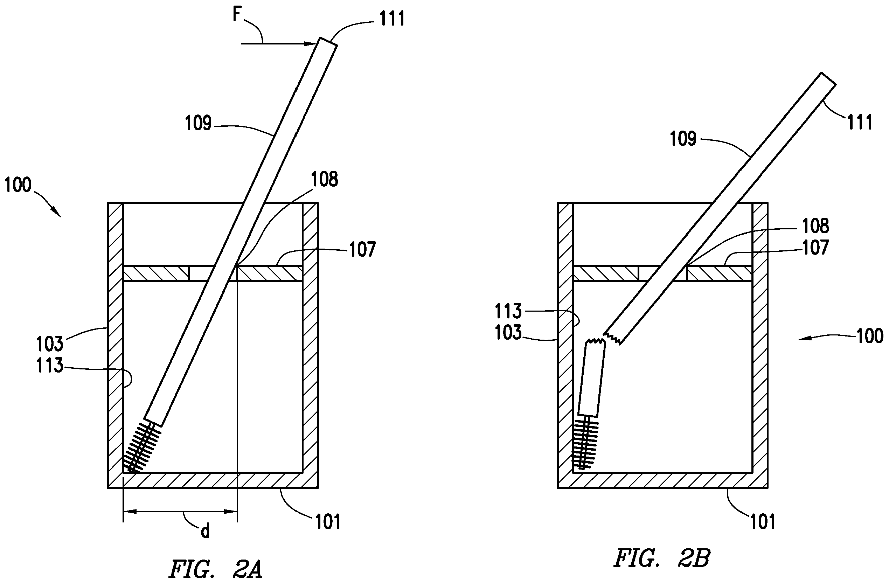

In some embodiments, tissue sample receptacle 100 may include fulcrum body 107. Fulcrum body 107 may be positioned within tissue sample receptacle 100. In some embodiments, fulcrum body 107 may, for example and without limitation, provide fulcrum 108 against which tissue sample collector 109 may be bent as depicted in FIGS. 2A, 2B. Fulcrum body 107 may be any structure that provides fulcrum 108. Tissue sample collector 109 may be broken by the application of force, depicted as force F, at top end 111 of tissue sample collector 109. Force F may bend tissue sample collector 109 between sidewall 103 and fulcrum 108 of fulcrum body 107. As understood in the art, as distance d between fulcrum body 107 and the side of sidewall 103 opposite fulcrum body 107 is reduced, the amount of force F required to break tissue sample collector 109 may be reduced. Although depicted as a brush, tissue sample collector 109 may be, for example and without limitation, a brush, spatula, or broom-like device.

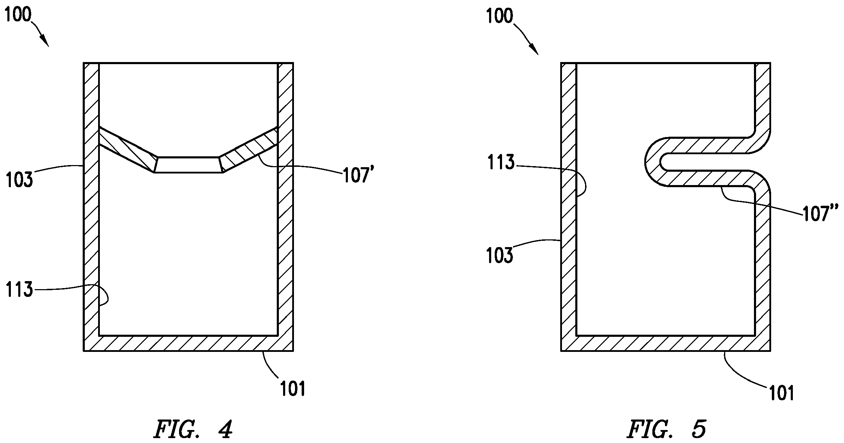

In some embodiments, fulcrum body 107 may be mechanically coupled to interior surface 113 of sidewall 103. In some embodiments, fulcrum body 107 may be coupled to bottom 101. In some embodiments, fulcrum body 107 may be coupled to an exterior surface of sidewall 103. In some embodiments, for example and without limitation, fulcrum body 107 may be mechanically coupled to or formed as part of cap 105. In some embodiments, as depicted in FIG. 3, fulcrum body 107 may be formed as a slotted disk through which tissue sample collector 109 may be extended prior to breaking. In some embodiments, as depicted in FIG. 4, fulcrum body 107' may be funnel shaped and coupled to interior surface 113 of sidewall 103.

In some embodiments, as depicted in FIG. 5, fulcrum body 107" may be a protrusion extending from interior surface 113 of sidewall 103. In some embodiments, fulcrum body 107" may be formed as an integral part of sidewall 103. In some such embodiments, fulcrum body 107" may be a molded extension into the interior of tissue sample receptacle 100. In some embodiments, fulcrum body 107" may be directly or indirectly attached to a surface or end of tissue sample receptacle 100.

In operation, with respect to FIGS. 2A, 2B, once a tissue sample has been obtained, tissue sample collector 109 may be inserted into tissue sample receptacle 100. Depending on the type of sample being collected, one or more tissue deposition operations may be performed, including, for example and without limitation, scraping, swirling, or otherwise manipulating tissue sample collector 109 within tissue sample receptacle 100. In some embodiments, a liquid may be contained within tissue sample receptacle 100. In some embodiments, tissue sample receptacle 100 may be manipulated into contact with sidewall 103 and fulcrum body 107 as depicted in FIG. 2A. Force may then be applied on top end 111 of tissue sample collector 109, denoted by force F until tissue sample collector 109 mechanically fails as depicted in FIG. 2B. In some embodiments, tissue sample collector 109 may include one or more stress concentration features adapted to facilitate easier and more predictable breakage of tissue sample collector 109. Stress concentration features may include, for example and without limitation, one or more grooves, ridges, cut outs, or holes formed in tissue sample collector 109. In some embodiments, cap 105 may be mechanically and sealingly coupled to sidewall 103 of tissue sample receptacle 100, sealing the broken-off portion of tissue sample collector 109 within tissue sample receptacle 100, allowing for safe storage or transport of the obtained tissue sample.

In some embodiments, fulcrum body 107 may be permanently mechanically coupled to tissue sample receptacle 100. In some such embodiments, fulcrum body 107 may be thermally, chemically, electrically, optically, frictionally, or beam-energy welded; press fit; or cemented in place to interior surface 113 of sidewall 103 as understood in the art. In some embodiments, fulcrum body 107 may be formed integrally with tissue sample receptacle 100. One having ordinary skill in the art with the benefit of this disclosure will understand that any suitable method for mechanically coupling fulcrum body 107 to tissue sample receptacle 100 may be utilized without deviating from the scope of this disclosure.

In some embodiments, fulcrum body 107 may be temporarily positionable within tissue sample receptacle 100. In such an embodiment, fulcrum body 107 may be positioned into an existing tissue sample receptacle 100 before tissue sample collector 109 is to be broken off therein. In some embodiments, fulcrum body 107 may be removed from tissue sample receptacle 100 after tissue sample collector 109 is broken off therein, allowing, for example and without limitation, access to the interior of tissue sample receptacle 100 below fulcrum body 107.

Although described herein with respect to tissue sample collection, the described container may be used for any purpose including any biological sampling or any other implementation in which a danger exists for contact with or handling of the samples.

The foregoing outlines features of several embodiments so that a person of ordinary skill in the art may better understand the aspects of the present disclosure. Such features may be replaced by any one of numerous equivalent alternatives, only some of which are disclosed herein. One of ordinary skill in the art should appreciate that they may readily use the present disclosure as a basis for designing or modifying other processes and structures for carrying out the same purposes and/or achieving the same advantages of the embodiments introduced herein. One of ordinary skill in the art should also realize that such equivalent constructions do not depart from the spirit and scope of the present disclosure and that they may make various changes, substitutions, and alterations herein without departing from the spirit and scope of the present disclosure.

The present invention is now described with reference to the following clauses:
1. A tissue sample receptacle comprising:
   a container; and
   a fulcrum body positioned on the interior of the container.
2. The tissue sample receptacle of clause 1, wherein the fulcrum body is a protrusion extending from an interior surface of a sidewall of the container.
3. The tissue sample receptacle of clause 1 or clause 2, wherein the fulcrum body is formed as an integral part of a sidewall of the container.
4. The tissue sample receptacle of any one of clauses 1 to 3, wherein the fulcrum body is coupled to an interior surface of a sidewall of the container.
5. The tissue sample receptacle of clause 4, wherein the fulcrum body is thermally, chemically, electrically, optically, frictionally, or beam-energy welded; press fit; or cemented to the interior surface of the sidewall.
6. The tissue sample receptacle of any one of clauses 1 to 5, wherein the fulcrum body is a slotted disk.
7. The tissue sample receptacle of any one of clauses 1 to 6, wherein the fulcrum body is funnel shaped.
8. The tissue sample receptacle of any one of clauses 1 to 7, further comprising a cap, the cap sealingly coupled to the container.
9. The tissue sample receptacle of any one of clauses 1 to 8, wherein the container is a bottle, vial, tube, or bag.
10. A method comprising:
   providing a tissue sample receptacle, the tissue sample receptacle including:
      a container; and
      a fulcrum body positioned on the interior of the container;
   inserting the tissue sample collector into the tissue sample receptacle;
   applying a force against an upper end of the tissue sample collector such that the tissue sample collector is bent between the fulcrum body and the container; and
   breaking the tissue sample collector.
11. The method of clause 10 further comprising removing the fulcrum body from the tissue sample receptacle.
12. The method of clause 10 or clause 1 l, further comprising inserting the fulcrum body into the tissue sample receptacle.
13. The method of clause 12, wherein the fulcrum body is coupled to an interior surface of a sidewall of the container.
14. The method of clause 13, further comprising thermally, chemically, electrically, optically, frictionally, or beam-energy welding; press fitting; or cementing the fulcrum body to the interior surface of the sidewall.
15. The method of clause 12, wherein the fulcrum body is formed as an integral part of a sidewall of the container.
16. The method of clause 12, wherein the fulcrum body is a protrusion extending from an interior surface of a sidewall of the container.
17. The method of any one of clauses 10 to 16, wherein the fulcrum body is a slotted disk.
18. The method of any one of clauses 10 to 17, wherein the fulcrum body is funnel shaped.
19. The method of any one of clauses 10 to 18, further comprising scraping, swirling, or otherwise manipulating the tissue sample collector within the tissue sample receptacle.
20. The method of any one of clauses 10 to 19, further comprising mechanically coupling a cap to the container.
21. The method of clause 20, wherein the cap is mechanically coupled to the container by a threaded connection or a snap fit.
22. The method of any one of clauses 10 to 21, further comprising prior to the step of inserting the tissue sample collector into the tissue sample receptacle:
   collecting a tissue sample with a tissue sample collector.

## Claims

1. A tissue sample receptacle comprising:
a container; and
a fulcrum body positioned on the interior of the container.

2. The tissue sample receptacle of claim 1, wherein the fulcrum body is a protrusion extending from an interior surface of a sidewall of the container.

3. The tissue sample receptacle of claim 1 or claim 2, wherein the fulcrum body is formed as an integral part of a sidewall of the container.

4. The tissue sample receptacle of any one of claims 1 to 3, wherein the fulcrum body is coupled to an interior surface of a sidewall of the container; optionally, wherein the fulcrum body is thermally, chemically, electrically, optically, frictionally, or beam-energy welded; press fit; or cemented to the interior surface of the sidewall.

5. The tissue sample receptacle of any one of claims 1 to 4, wherein the fulcrum body:
is a slotted disk; and/or,
is funnel shaped.

6. The tissue sample receptacle of any one of claims 1 to 5, further comprising a cap, the cap sealingly coupled to the container.

7. The tissue sample receptacle of any one of claims 1 to 6, wherein the container is a bottle, vial, tube, or bag.

8. A method comprising:
providing a tissue sample receptacle, the tissue sample receptacle including:
a container; and
a fulcrum body positioned on the interior of the container;
inserting the tissue sample collector into the tissue sample receptacle;
applying a force against an upper end of the tissue sample collector such that the tissue sample collector is bent between the fulcrum body and the container; and
breaking the tissue sample collector.

9. The method of claim 8, further comprising removing the fulcrum body from the tissue sample receptacle.

10. The method of claim 8 or claim 9, further comprising inserting the fulcrum body into the tissue sample receptacle.

11. The method of claim 10, wherein the fulcrum body is coupled to an interior surface of a sidewall of the container; optionally, further comprising thermally, chemically, electrically, optically, frictionally, or beam-energy welding; press fitting; or cementing the fulcrum body to the interior surface of the sidewall.

12. The method of claim 10, wherein the fulcrum body:
is formed as an integral part of a sidewall of the container; and/or,
is a protrusion extending from an interior surface of a sidewall of the container.

13. The method of any one of claims 8 to 12, wherein the fulcrum body:
is a slotted disk; and/or,
is funnel shaped.

14. The method of any one of claims 8 to 13, further comprising:
scraping, swirling, or otherwise manipulating the tissue sample collector within the tissue sample receptacle; and/or,
mechanically coupling a cap to the container; optionally, wherein the cap is mechanically coupled to the container by a threaded connection or a snap fit.

15. The method of any one of claims 8 to 14, further comprising prior to the step of inserting the tissue sample collector into the tissue sample receptacle:
collecting a tissue sample with a tissue sample collector.
